# EUROPEAN PATENT SPECIFICATION

(11) **EP 1 446 144 B1**
(45) Date of publication and mention of the grant of the patent: **09.07.2008**
(21) Application number: 02788489.9
(22) Date of filing: 21.11.2002
(51) Int. Cl.: A61K 38/22, A61P 15/00

(54) **THE USE OF LEPTIN IN INFERTILITY**
VERWENDUNG VON LEPTIN BEI UNFRUCHTBARKEIT
UTILISATION DE LA LEPTINE DANS LE TRAITEMENT DE L'INFERTILITE

(30) Priority: 21.11.2001 IL 14665501
(43) Date of publication of application: 18.08.2004
(73) Proprietor: YEDA RESEARCH AND DEVELOPMENT CO., LTD., 76100 Rehovot (IL)
(72) Inventor: Barkan, Dalit, Rockville, MD 20852 (US); DEKEL, Nava, Weizmann Institute, 76100 Rehovot (IL); RUBINSTEIN, Menachem, 76329 Rehovot (IL); HURGIN, Vladimir, 77223 Ashdod (IL)
(74) Representative: Weiss, Wolfgang
(86) International application number: PCT/IL2002/000928
(87) International publication number: WO 2003/043652

(56) References cited:
- WO-A-98/36763
- B. ALMOG ET AL.: "Leptin attenuates follicular apoptosis and accelerates the onset of puberty in immature rats." MOLECULAR AND CELLULAR ENDOCRINOLOGY, vol. 183, no. 1-2, 25 October 2001 (2001-10-25), pages 179-191, XP002243356
- CHEHAB F F ET AL: "CORRECTION OF THE STERILITY DEFECT IN HOMOZYGOUS OBESE FEMALE MICE BY TREATMENT WITH THE HUMAN RECOMBINANT LEPTIN" NATURE GENETICS, NEW YORK, NY, US, vol. 12, 1 March 1996 (1996-03-01), pages 318-320, XP002060725 ISSN: 1061-4036 cited in the application
- S GREISEN ET AL.: "Effects of leptin on basal and FSH stimulated steroidogenesis in human granulosa luteal cells." ACTA OBSTETRICIA ET GYNECOLOGICA SCANDINAVICA, vol. 79, no. 11, November 2000 (2000-11), pages 931-935, XP002243357 cited in the application
- I. E. MESSINIS ET AL.: "Leptin in human reproduction." HUMAN REPRODUCTION UPDATE, vol. 5, no. 1, 1999, pages 52-63, XP008017998
- J.D. BRANNIAN ET AL.: "Leptin and ovarian folliculogenesis: Implications for ovulation induction and ART outcomes." SEMINARS IN REPRODUCTIVE MEDICINE, vol. 20, no. 2, May 2002 (2002-05), pages 103-112, XP008018034

## Description

### FIELD OF THE INVENTION

The present invention relates to the use of leptin in treating infertility for triggering ovulation.

### BACKGROUND OF THE INVENTION

The ovary is a ductless gland of the female in which the ova (female reproductive cells) are produced. In vertebrate animals the ovary also secretes the sex hormones oestrogen and progesterone, which control the development of the sexual organs and the secondary sexual characteristics. In humans, the interaction between the gonadotropic hormones from the pituitary gland and the sex hormones from the ovary controls the monthly cycle of ovulation and menstruation. About 500,000 immature eggs are present in the cortex of the ovary at birth. Starting at puberty, eggs mature successively, and one breaks through the ovarian wall about every 28 days in the process known as ovulation, which continues until menopause, or cessation of reproductive functioning in the female. After its release from the ovary, the ovum passes into the oviduct (uterine or fallopian tube) and into the uterus. If the ovum is fertilized by the sperm, pregnancy ensues.

The ovary consists of a cortical zone composed of a specialized stroma, which contains follicles with ova. In the mature functional ovary, many follicles are quiescent, whereas others exhibit a wide range of histomorphology, depending upon their stage of maturation or regression. The medulla consists primarily of connective tissue and an extremely rich vascular supply.

During human fetal development, the primordial germ cells migrate to and are incorporated within the developing ovary and are termed *oogonia.* The oogonia multiply by mitosis, but early in fetal life, they enter meiosis. However, the meiotic events are arrested by a mechanism not understood in prophase (diplotene stage) of the first meiotic division. These cells, about 40 µm in diameter and termed *primary oocytes,* are enclosed within a single layer of squamous cells, forming a primordial follicle.

The transition from an inactive primordial follicle to a growing and maturing primary follicle involves changes in the oocyte, the follicular cells, and the adjacent connective tissue. As the oocyte enlarges, the single layer of follicular cells increases in size through mitotic division and gives rise to cells (granulosa cells) that eventually form a stratified epithelium termed the *granulosa*. A distinctive feature of the multilaminar follicle is the elaboration of a highly refractive zona pellucida interposed between the oocyte and granulosa cells; the zona is secreted by both the egg and surrounding follicular cells. Concomitant with the development of the granulosa cells, a sheath of stromal cells (theca folliculi) develops around the follicle and subsequently forms two layers. The inner layer exhibits a well-developed capillary plexus and secretory cells and is termed the *theca interna*. The cells of the theca interna are believed to secrete androstenedione, which is subsequently converted to estradiol by the granulosa cells. Secondary follicles can be identified when they are about 0.2 mm in diameter and are recognized by the presence of irregular spaces among the granulosa cells filled with a clear liquid (liquor folliculi), which increases with continued growth of the follicle. Eventually, the oocyte comes to be eccentrically placed within the follicle upon a pedestal of follicular cells, the cumulus oophorus. The oocyte is intimately surrounded by a crown of follicular cells, the corona radiata. The cumulus projects into a single large fluid-filled space, the antrum, formed from the coalescence of the smaller spaces noted previously.

Even after the primary oocyte has reached full size, the follicle may continue to enlarge until it reaches approximately 10 mm in diameter. Follicles that have matured to maximal size, exhibit a large antrum, and extend through the entire thickness of the cortex are termed *graafian follicles.* Just prior to ovulation, a bulge on the surface of the ovary (the stigma) marks the site where ovulation will occur. The growth of a primordial follicle to full maturity takes about 10 to 14 days. The thecae folliculi, particularly the theca interna, reach their highest development in relation to the mature follicle.

At mid-menstrual cycle (approximately day 14), a surge of pituitary luteinizing hormone (LH) induces ovulation. At this time, the primary oocyte's first meiotic division occurs, resulting in the formation of the first polar body and the secondary oocyte.

Following ovulation and discharge of the liquor folliculi and the oocyte within its cumulus mass, the walls of the follicle collapse and the granulosa cell lining becomes folded. Rupture of blood vessels in the theca interna is associated with bleeding into the partially collapsed follicle, and a clot is formed. The cells of the granulosa layer and the theca interna undergo transformation and are renamed *granulosa lutein* and *theca lutein cells,* respectively. These changes in the follicle following ovulation result in a new but transitory organ, the corpus luteum (yellow body, for its appearance in fresh specimens). The corpus luteum secretes the hormone progesterone. If the ovulated oocyte fails to be fertilized, the corpus luteum remains functional for only about 14 days and then regresses and is reduced eventually to a scar within the ovary termed the *corpus albicans* (white body). In the event of fertilization, the corpus luteum enlarges and persists as a functional endocrine gland throughout most of the pregnancy but begins to involute after the sixth month. Its ultimate fate after the termination of pregnancy is to become a large corpus albicans.

In mammals, the process of ovarian follicle maturation, ovulation, corpus luteum formation, and finally its dissolution, are repeated at each oestrus and menstrual cycle. These processes are initiated with the pre-ovulatory increase in gonadotropic hormones: follicle-stimulating hormone (FSH) and luteinizing hormone (LH). FSH stimulates the development of ovarian follicles and steroidogenesis, while LH induces maturation of the oocyte by resumption of meiosis, accompanied by an ovulation and luteinization of granulosa and theca cells to form the corpus luteum [Amsterdam et al. 1987]. Gonadotropin-induced follicle maturation is accompanied by a sharp increase in blood-progesterone to prepare the uterus for possible ovum implantation.

LH and FSH are secreted by the pituitary and together play a central role in regulating the menstrual cycle and ovulation and hCG is secreted by the developing placenta from the early stages of pregnancy and its role is to maintain steroid secretion by the corpus luteum, which is necessary to prevent ovulation during pregnancy.

In the normal cycle, there is a mid-cycle surge in LH concentration, which is followed by ovulation. An elevated oestrogen level, which is brought about by the endogenous secretion of LH and FSH, is required for the LH surge to occur. The oestrogen mediates a positive feedback mechanism, which results in the increased LH secretion.

At the beginning of each ovulatory cycle, the FSH signal from the anterior pituitary gland stimulates a small amount of ovarian follicles to grow. These follicles are endowed with receptors for FSH and are not responsive to LH at this time as they do not posses LH receptors (Chappel et al.1991). These follicles respond to the initial stimulation of FSH by cell growth and proliferation. FSH stimulates the transcription of genes that encode growth factors. These factors act in a paracrine and autocrine way within the follicular microenvironment. They play an important role in the final maturation of the follicle and its contents. During the mid-follicular phase, following FSH stimulation, the granulosa cells begin to synthesize LH receptors and acquire the ability to respond to the stimulatory effects of LH (Simon et al. 1988).

LH stimulation of specific cells within the ovary is required for the production of steroid hormones. As described by the two-cell theory, LH is critical for the production of androgens from the interstitial theca cells. Androgens produced by theca cells travel to the granulosa cells to be converted to estradiol. During the early follicular phase, LH receptors are found only on ovarian theca cells (Erickson et al. 1979). Following LH stimulation, the theca cells express a series of enzymes that convert cholesterol to androgens. Androgens are converted to estrogens within the FSH-stimulated granulosa cells (Remohi et al. 1996/1997).

There is no debate, however, about the importance of the mid-cycle release of LH: it is a requirement for ovulation of a fertile ovum. This preovulatory surge of LH initiates not only events within the ova (resumption of meiosis) but also activation of enzymes that weaken the follicular wall to facilitate the extrusion of follicular contents. LH surge is associated with transcriptional regulation of numerous genes, and is presumed to involve the synthesis and/or activation of specific proteases that degrade the follicle wall. The progesterone receptor, a nuclear receptor transcription factor, is induced in granulose cells of pre-ovulatory follicles in response to the LH surge and has been shown to be essential for ovulation, because mice lacking progesterone receptors fail to ovulate and are infertile. In an animal model it has been demonstrated that two proteases, ADAMTS-1 (A disintegrin and metalloproteinase with thrombospondin-like motifs) and cathepsin L (a lysosomal cysteine protease) are transcriptional targets of progesterone receptor action and therefore are probably involved in degradation of the follicle wall, (Robker et al. 2000).

Elevated levels of serum LH during the follicular phase of the menstrual cycle are not only unnecessary for follicular maturation but are deleterious to a normal reproductive process (Remohi et al. 1996/1997). These elevations may occur as a result of administration of exogenous LH or through an endogenous pathological process (ex. policystic ovary) (Remohi et al. 1996/1997).

The re-evaluation of the two cell-two gonadotrophin theory suggests that during the preovulatory period, resting levels of LH are adequate for normal follicular maturation. Indeed, overstimulation of the ovary with excessive amounts of LH may diminish the ability of that target organ to produce fertile ova (Schoot et al. 1994).

LH is important for the production of estrogens by stimulating the theca cells to produce androgens and then estradiol. The formation of LH receptors is induced by FSH in the granulosa cells of the dominant follicle and with this the importance for it (the follicle) to maintain its growth when FSH levels decline. The follicles that have LH receptors are the only ones who are going to be able to respond to the LH pulse that activates the mechanisms that sparks ovulation.

In several studies of ovarian stimulation with RecFSH, normal follicular growth was induced but with low remaining levels of estradiol and androstenedione concentrations (Dick et al.1992, Mannaerts et al. 1996, Schoot et al. 1994).

This indicates that ovarian follicles are incapable of producing sufficient amounts of androstenedione (AD) in the presence of minute amounts of LH (bellow 0.38 IU/L) (Mannaerts et al. 1996, Schoot et al. 1994). The subsequent inability of normal estrogen production within follicles is in keeping with the two cell - two gonadotrophin hypothesis, indicating that FSH induced granulosa cell aromatase activity can only lead to augmented estradiol production if a sufficient amount of the aromatase substrate androstenedione is available (Schoot et al.1994, Kessel et al.1985).

In order with this, stimulation experiments in rats with human recombinant FSH (hRecFSH) and human recombinant LH (hRecLH), resulted in increased ovarian estrogen secretion that only occurred if both hRecFSH and hRecLH were given simultaneously (Smyth et al. 1995). Treatment with hRecFSH alone stimulated the granulosa cell aromatase activity without estrogen secretion, whereas hRecLH alone stimulated the thecal androgen synthesis and androgen secretion (Smyth et al. 1995).

This underlines the concept of a LH threshold for sufficient estrogen production and for an optimal induction, thinking that exposure to hRecLH may improve embryo viability and the rate of development (Weston et al. 1996).

For the majority of patients for whom FSH therapy is indicated, LH administration is not required to achieve follicular development as sufficient endogenous LH is present (as shown in women with WHO group II anovulation (Schoot et al. 1994) and patients stimulated for Assisted Reproductive Technologies). In contrast, the majority of women with hypogonadotropic hypogonadism (WHO group I anovulation) do not have the threshold levels of endogenous LH required to achieve optimal follicular development and steroidogenesis during therapy with FSH alone. Among these women, urinary and RecFSH have been shown to achieve considerably lower estradiol levels than that obtained with HMG preparations containing both, FSH and LH (Hillier et al. 1991). It also appears that in this population, the follicles stimulated by FSH alone do not consistently rupture after hCG administration. They luteinize poorly and oocytes may have a lower fertilization rate (Hillier et al. 1991). An exogenous supply of LH is required to achieve an adequate follicular response.

The same study (Schoot et al. 1994),) also confirms when FSH alone is used to stimulate follicular development, follicular growth occurs, but estradiol secretion is minimal, resulting in deficient endometrial growth. In addition, when exposed to hCG, these follicles frequently fail to luteinize (Hillier et al. 1991).

The successful induction of ovulation in women with HH and intact pituitary function has been achieved with pulsatile GnRH therapy. Pulsatile GnRH therapy has been the treatment of choice because it restores the pulsatile released gonadotrophins from the pituitary. This results in predominantly unifollicular cycles and satisfactory pregnancy rates. The treatment is associated with low rates of multiple pregnancies and is not complicated by ovarian hyperstimulation syndrome (The European Recombinant Human LH Study Group 1998).

However, for patients who do not respond adequately to pulsatile GnRH or those with pituitary disease, HMG therapy (preparations containing both, FSH and LH) administered as a once daily injection has been the only alternative treatment for ovulation induction.

Different methods are used to correct or circumvent the many different functional disorders of females that can interfere with conception and childbearing.

The most common cause of female infertility is failure to ovulate. In certain cases this can be corrected with the drug clomiphene citrate (Clomid, Serophene). Introduced in 1967, clomiphene stimulates the release of the gonadotropic hormones: follicle-stimulating hormone (FSH) and luteinizing hormone (LH). FSH functions to stimulate the ovarian follicle (the egg and its surrounding fluid and hormones); LH triggers ovulation. In some studies, clomiphene has been associated with an increased risk of ovarian cancer.

Human menopausal gonadotropin, or menotropin (Pergonal), introduced in 1970, is an extract from the urine of menopausal women. It contains FSH and LH and encourages ovulation. It is often given together with human chorionic gonadotropin (HCG), a hormone secreted by the placenta during pregnancy and obtained from the urine of pregnant women. Its action is similar to that of luteinizing hormone.

Urofollitropin (Metrodin) is essentially FSH without LH. It is used especially in women with polycystic ovary syndrome, who tend to have too little FSH and too much LH.

Progesterone is a female sex hormone that induces secretory changes in the lining of the uterus essential for successful implantation of a fertilized egg. It is liberated by the ovary after the ovum is released. It is administered in cases where fertilization of the ovum does occur but where there is evidence that the uterine lining is unable to support the developing fetus, as in repeated miscarriages or bleeding during pregnancy.

There are several procedures designed to unite sperm and eggs, which bypass altogether some of the factors causing infertility. Collectively, these procedures are referred to as assisted reproductive technologies (ART). Although most couples do not require these procedures to conceive, ARTs are available for those who do not respond to other therapies.

ART procedures involve the use of various hormones to stimulate the growth of as many oocytes as possible. This multiple oocyte development increases the chances for fertilization and, pregnancy. The most common ART procedures are:
1- In vitro fertilization (IVF)
2- Intracytoplasmic Sperm Injection (ICSI)
3- Gamete intrafallopian transfer (GIFT)

IVF was originally developed to treat infertility caused by blocked or damaged fallopian tubes. It is currently used to treat a variety of infertility problems.

IVF involves collecting eggs and sperm from a couple and placing them together in a laboratory environment. Usually up to three fertilized eggs, or embryos, are then transferred into the woman's uterus, or womb, where implantation and embryo development will hopefully occur just as in a normal pregnancy.

In vitro fertilization (IVF) is a four-stage procedure:
Stage 1 - Ovarian stimulation and monitoring
Stage 2 - Egg (oocyte) retrieval
Stage 3 - Fertilization
Stage 4 - Embryo transfer

In order to maximize the chances for successful fertilization with each IVF attempt, ovarian stimulation is used to produce multiple mature follicles, rather than the single egg normally developed each month. By having several mature eggs available for fertilization and transfer, the chances of fertilization and pregnancy are increased.

Ovarian stimulation involves the use of follicle-stimulating hormone (FSH), the hormone necessary for multiple oocyte development. FSH is available in three forms: a FSH/LH combination ratio of one to one; highly purified FSH with almost no LH, introduced in Canada in 1994; and recombinant FSH available since 1997.

FSH is given by daily injection. A response to the treatment is generally evident after five to seven days. The number of days and the dose will vary depending on follicle development. The physician monitors the response to the treatment and time the administration of a second product, human chorionic gonadotropin for injection, which triggers ovulation in women, and is usually administered after the last dose of FSH.

Ovarian stimulation and ART may start by "down regulation" or "pituitary desensitization". This is the medical suppression of normal function of the pituitary gland (which normally produces FSH to stimulate a single follicle to develop, and LH to trigger ovulation in an unstimulated cycle). To achieve this control, a drug may be prescribed to begin taking several days before beginning FSH therapy. A blood test will determine when pituitary desensitization has occurred and when to begin FSH therapy. The use of suppression increases the yield of mature eggs and prevents the body from releasing them before they can be retrieved or collected. The suppression medication will be taken throughout the FSH therapy.

A series of ultrasound scans will be carried out to monitor follicle growth in the ovary. As follicles mature, they grow larger. Through ultrasound, follicle growth, number and size, can be followed allowing to make any necessary adjustments in the daily dose and to determine follicle maturity and the ideal time for hCG administration.

Developing follicles secrete increasing amounts of the female hormone estrogen, particularly estradiol (E2). Together with ultrasound, E2 levels may be used to determine the optimal timing for the administration of hCG.

The next stage of therapy is called egg recovery or retrieval. Once hCG is administered, as many mature eggs as possible will be retrieved. Ultrasound-guided aspiration (e.g. vacuum suction) is now the most common method for egg retrieval. Egg recovery is accomplished by a transvaginal procedure, which can be performed under light sedation or local anesthesia. The ultrasound image allows for accurate aspiration of the follicle. Eggs are then identified under the microscope by the embryologist and transferred to a carefully controlled environment prior to fertilization.

Shortly before the eggs are retrieved, a semen sample is collected by the male partner (or donor) and processed using various laboratory techniques. The goal is to obtain the strongest, most active sperm from the ejaculate through sperm washing.

Once mature eggs have been retrieved, the sperm and eggs are placed together in the laboratory and incubated in a meticulously-prepared culture medium at a temperature identical to that of the woman's body. After approximately 48 hours, if the eggs have been successfully fertilized and are growing normally, they are ready to be transferred to the uterus.

Usually up to three embryos ate placed together and transferred, via the vagina, into the uterus. Any high quality embryos that have not been transferred can be frozen (or cryopreserved) and stored for future use.

Women treated with human menopausal gonadotropins, as a prelude to ovulation or follicle aspiration for oocyte collection in vitro fertilization techniques (IVF), often fail to demonstrate a timely LH surge despite serum estradiol levels sufficient to elicit positive feedback of LH secretion. Usually hCG is used in place of LH to induce ovulation. One of the problems of Ovarian Hyperstimulation Syndrome is the administration of the hCG dose to achieve ovulation (Hiller et al.1995).

Individual responses to human menopausal gonadotropins vary markedly, thereby complicating patient management even when the most flexible (individualized) protocols are used.

Leptin is an adipocyte-secreted protein, encoded by the obese (ob) gene, responsible for the phenotype of obesity, diabetes and insulin resistance in *ob*/*ob* mice (Zhang et al. 1994). The gene is highly conserved, exhibiting 84% identity with the mouse protein (Zhang et al. 1994). An assay for plasma leptin (Considine et al. 1996) revealed tight correlation between body mass index and plasma leptin, in obese individuals, suggesting that the most frequent form of human obesity is associated with a phenomenon of leptin resistance.

In addition to its metabolic functions, leptin plays a critical role in reproduction. The obese leptin-deficient *ob*/*ob* mice serve as a rodent model for hypogonadism and unovulation. Their infertility is due to hypothalamic-pituitary hormone insufficiency and it can be rescued by administration of recombinant leptin [Barash et al. 1996, Mounzih et al. 1997, Hwa et al. 1997, Muzzin et al. 1996, Chehab et al. 1996 and Schwartz et al. 1996]. Congenital leptin deficiency in humans was also reported and found to be associated with hypogonadotrophic hypogonadism [Strobel et al.1998 and Farooqi et al.1999].

As to leptin's mode of action, it was found that binding of leptin to its hypothalamic receptor is required for release of gonadotropin releasing hormone (GnRH), which subsequently induces the synthesis and release of pituitary FSH and LH [Nagatani et al. 1998]. Furthermore, serum leptin rises during the follicular phase to reach a peak at the luteal phase of the spontaneous cycle, strongly suggesting a role for leptin in ovulation [Hardie et al. 1997, Shimizu et al. 1997 and Messinis et al. 1998]. Yet, the role of leptin in reproduction is not fully understood (Ahima et al. 1996, Erickson et al. 1996 and Yu et al. 1997) and results reported show the possibility of the direct action of leptin on ovary cells. In one study, the leptin receptor was shown to be expressed in the human ovary, leptin was found in follicular fluid, and was reported to suppress LH-induced estradiol production in primary cultures of human granulosa cells indicating that leptin can exert biological effect on granulosa cells (Karlsson et al. 1997). In another study concerning IVF procedures, serum leptin levels were monitored during an ovarian suppression-stimulation programme. Leptin concentration decreased significantly from the normal mid-luteal phase to ovarian suppression and rose significantly following ovarian stimulation by HMG/FSH, regardless of body mass index. These alterations in leptin may be caused by the parallel changes in oestrogenic milieu. The relationship between the rise in oestradiol and leptin during ovarian stimulation may be an important factor for successful IVF outcome. In addition a successful outcome of pregnancy was associated with high concentrations of leptin at 12 days after embryo transfer (Unkila-kallio et al 2001).

The above findings are consistent with an endocrine beneficial action of leptin in ovulation and fertility. In contrast, the following findings indicate a negative action of leptin in ovulation and fertility.

In vitro treatment of granulosa cells (GS) with FSH results in stimulation of E2 and P4 production. IGF-I is a sensitising factor that has been proposed to synergise the FSH activity, and thus in the presence of IGF-I, the FSH-dependent E2 levels are augmented. This study has shown that leptin can act directly on the ovarian GC to selective decrease E2, but not P4 production. FSH-dependent E2 production was not reduced by leptin whereas leptin impaired the sensitising effect of IGF-1 on FSH-dependent E2 synthesis by GC (Zachow et al. 1997). A direct effect of leptin in ovarian steroid production was shown by Barkan et al. (1999). It was shown that leptin suppressed ovarian steroid synthesis costimulated by FSH an dexamethasone and costimulation of progesterone production by forskolin and dexamethasone. In a similar experimental setting it was found by Greisen et al. (2000) that leptin inhibits basal and FSH stimulated estradiol and progesterone production in cultured granulosa cells.

The in vivo level of leptin in patients receiving Clomiphene Citrate therapy for induction of ovulation was found to be lower in patients who remain anovulatory (Imani et al. 2000).

The in vivo level of leptin in patients receiving gonadotropic therapy for induction of ovulation was monitored. It was found that progressive decline in pregnancy rate correlated with increased serum leptin over body mass index ratio (Brannian et al. 2001). In view of these results it was suggested that woman might be able to improve their fertility through dietary and lifestyle modifications that lower circulating leptin concentrations.

WO 98/36763 refers to the treatment of infertility with leptin receptor ligands or with antibodies to leptin receptor agonists.

Bruneau et al. (médicine/sciences 1999; 15: 191-196) is an overview on the role of leptin in reproduction.

Although a clear explanation for these apparent discrepant observations is not obvious, differential actions may result from contribution of targeted stimulation of the ovary versus that of indirect, secondary effects such as general changes in metabolic state, weight-loss, circulating insulin levels etc. The timing of leptin administration and/or leptin measurements in the circulation could also influence the results obtained by different groups.

For obvious ethical reasons, only minimal, directly derived scientific information exists on the impact of ovarian stimulation on follicle development, implantation and gestation in humans. Therefore, for the majority of such studies, rodents are the preferable models. Considerable similarities exist among many mammalian species in the early stages of development with respect to morphology and metabolism. This suggests that information obtained on oocyte development from laboratory animals might be applicable to those of the human subjects.

Therefore exists a need to provide a method to support ovulation in women which are not responsive to LH and hCG treatment or in which administration of these gonadotropins increase the risk of ovarian hyperstimulation syndrome or polycistic ovary syndrome. This invention discloses the use of leptin for increasing the likelihood of ovulation despite the above-mentioned contradictory considerations regarding the involvement of leptin in ovulation.

### SUMMARY OF THE INVENTION

The invention relates to the use of leptin, a fused protein, or functional fragment thereof in the manufacture of a medicament for treatment of female infertility, to trigger ovulation, follicular development, lutenization and follicular rupture.

In particular, the invention relates to the use of leptin, a fused protein, or functional fragment thereof in females that are not responsive to treatment with a fertility drug.

The invention relates also to the use of leptin, a fused protein, or functional fragment thereof in a female in which the administration of LH and hCG increases her risk of acquiring ovarian hyperstimulation syndrome or polycistic ovary syndrome or the use of leptin, a fused protein, or functional fragment thereof in a female with pituitary disease.

Together with leptin, a fused protein, or functional fragment thereof, a fertility drug such as FSH, GnRh, Clomiphene citrate, HMG and progesterone may be administered in accordance with the invention.

In addition, leptin, a fused protein, or functional fragment thereof may be used for treatment in assisted reproductive technologies such as in-vitro fertilization.

### BRIEF DESCRIPTION OF THE FIGURES

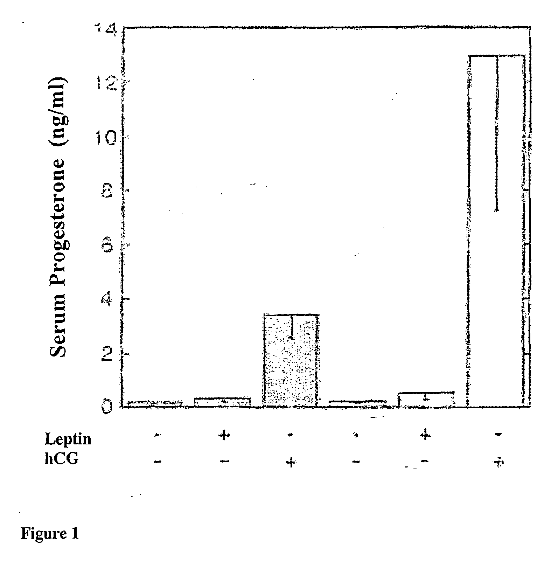

Figure 1 shows serum progesterone in *ob*/*ob* and C57BL/6 mice. Mice were treated with PMSG at time 0 and the indicated agent at 48 h. Progesterone was determined by RIA at 72 h in sera of C57BL/6 mice (grey bars) and of *ob*/*ob* mice (open bars).

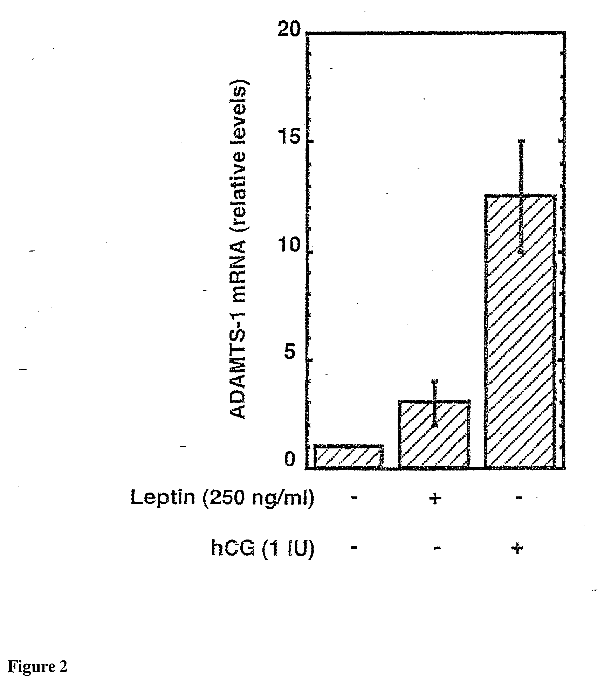

Figure 2 shows that leptin induces protease expression in preovulatory follicles. Pre-ovulatory follicles were removed cultured and further treated for 17 h in the absence (c) or presence of either leptin (250 ng/ml) or hCG (1 IU). Total RNA was isolated from the follicles and subjected to quantitative RT-PCR with specific primers for two proteases, ADAMTS-1 and Cathepsin L. The PCR products were resolved in agarose gel electrophoresis and scanned. The RNAs levels of ADAMTS-1 were normalized to the RNAs levels of ribosomal protein

### DETAILED DESCRIPTION OF THE INVENTION

The present invention relates to the administration of leptin, a fused protein, or functional fragment thereof, alone or in combination with fertility drugs for inducing ovulation in the treatment of female infertility. Infertility is defined as the inability to conceive after a year of regular intercourse without contraception. Female infertility is the term used when the infertility derives from a condition in the woman rather than the man.

The present invention is based in the finding that leptin triggers follicle development and ovulation directly, through a GnRH-independent pathway in antide treated *ob*/*ob* mice, a rodent model for hypogonadism and unovulation, and in prepubertal C57BL/6 mice.

In an embodiment of the invention it has been shown that administration of leptin into antide treated mice, which are unable to release LH from the pituitary and therefore exhibit very low levels of circulating LH, mediates follicular development. Moreover, follicular development in the hypogonadal *ob*/*ob* mice was very rapid and large antral follicles were seen as early as 9 h after leptin administration. Thus, leptin triggers follicular development by a GnRH-independent pathway.

In another embodiement leptin was shown to substitute the activity of hCG, the mimic of LH, in inducing ovulation. More specifically, ovulation was studied in ob/ob mice and in prepubertal C57BL/6 mice. All mice were treated with antide to prevent induction of LH by leptin. Ovulation was achieved in control mice by a combination of a non-super-ovulatory dose of PMSG and hCG. In a second group of mice, hCG was replaced by leptin. Following the various treatments, mice were sacrificed and oocytes were collected from the ampoule at 72 hours from the first injection and counted. Also the formation of corpora lutea, a marker of ovulation, was examined by staining of ovarian sections.

The results demonstrated that ovulation was also observed in mice in which hCG was replaced by leptin. Thus, leptin is able to replace hCG as inducer of ovulation in both *ob*/*ob* and in normal premature C57B1 mice.

Therefore, in addition of inducing follicular growth (see Example 1), leptin, like hCG and LH, directly triggers ovulation. Therefore leptin, a fused protein, or functional fragment thereof, can be used as a substitute of LH and hCG for inducing follicle development and/or ovulation in the treatment of female infertility.

LH surge is associated with transcriptional regulation of numerous genes, and is presumed to involve the synthesis and/or activation of specific proteases that degrade the follicle wall. The progesterone receptor, a nuclear receptor transcription factor, is induced in granulosa cells of pre-ovulatory follicles in response to the LH surge. It has been demonstrated that two proteases, ADAMTS-1 (A disintegrin and metalloproteinase with thrombospondin-like motifs) and cathepsin L (a lysosomal cysteine protease) are transcriptional targets of progesterone receptor action and therefore are probably involved in degradation of the follicular wall, (Robker et al. 2000). In one embodiment of this invention, it has been demonstrated that leptin induces directly the expression of ADAMTS-1 in pre-ovulatory follicles, indicating that leptin, like LH, has a role in the rupture of the follicle. Therefore leptin, a fused protein, or functional fragment thereof can be used to obtain follicle rupture instead of LH.

Therefore, the present invention relates to the use of leptin, a fused protein, or functional fragments thereof for the treatment of female infertility and more specifically to the use of leptin, a fused protein, or functional fragments thereof to support follicle development and/or to induce ovulation and/or to induce follicle rupture and/or to induce leutenization. Leptin, a fused protein, or functional fragment thereof can be administrated for the treatment of female infertility alone or in combination with fertility a drug such as hCG, LH, GnRH, Clomiphene citrate, HMG, and progesterone.

Ovulation is the release of a mature egg from an ovary during the menstrual cycle.

The term "fused protein" refers to a polypeptide comprising an leptin, or fragment thereof, fused with another protein, which, e.g., has an extended residence time in body fluids. A leptin , or functional fragment thereof may thus be fused to another protein, polypeptide or the like, e.g., an immunoglobulin or a fragment thereof.

As "Fragment " of a leptin, or fused protein, thereof of the present invention covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has substantially similar activity to leptin.

The term *fertility drug* primarily refers to drugs that mimic or stimulate production of a hormone necessary for conception, but it may also be used to refer to the hormones themselves, when they are administered as part of a program of infertility treatment. The following are commonly used fertility drugs:
Clomiphene citrate (Clomid, Serophene) stimulates the release of the gonadotropic hormones: follicle-stimulating hormone (FSH) and luteinizing hormone (LH).
FSH functions to stimulate the ovarian follicle (the egg and its surrounding fluid and hormones).
LH triggers ovulation.
GnRH induces FSH and LH release from the pituitary.
Chorionic gonadotropin (hCG) is often given together with FSH and LH. Its action is similar to that of luteinizing hormone.
HMG: preparations containing both, FSH and LH.
Progesterone is a female sex hormone that induces secretory changes in the lining of the uterus essential for successful implantation of a fertilized egg. It is released by the ovary after the ovum is released. It is administered in cases where fertilization of the ovum does occur but where there is evidence that the uterine lining is unable to support the developing fetus, as in repeated miscarriages or bleeding during pregnancy.

One of the problems of hCG administration is that it has a tendency to trigger Ovarian Hyperstimulation Syndrome. Therefore another important consideration for the use of leptin, a fused protein, or functional fragment thereof instead of hCG would be for patients with high risk of Ovarian Hyperstimulation Syndrome.
Women may be treated with leptin, a fused protein, or functional fragment thereof alone or in combination with fertility drugs as a prelude to ovulation or follicle aspiration in order to obtain oocytes for in vitro fertilization (IVF). The advantage of this method is to overcome often lack of a timely LH surge despite serum estradiol levels.

Expression of LH receptors is induced by FSH in the granulosa cells. The follicles that have LH receptors are the only ones who are going to be able to respond to the LH pulse that activates the mechanisms that sparks ovulation.

Leptin, a fused protein, or functional fragment thereof alone or in combination with fertility drugs could be used to support ovulation of patients insensitive to LH due to the lack of or impaired LH receptor.

Leptin, a fused protein, or functional fragment thereof alone or in combination with fertility drugs can be administrated at different stages during the menstrual cycle to support ovulation instead of LH in such cases when administration of LH is harmful, for example, when elevated levels of serum LH are already found during the follicular phase as a result of pathological process such as polycistic ovary syndrome. For the majority of patients for whom FSH therapy is indicated, LH administration is not required to achieve follicular development, as sufficient endogenous LH is present (as shown in women with WHO group II anovulation (Schoot et al. 1994) and patients stimulated for Assisted Reproductive Technologies). In contrast, the majority of women with hypogonadotropic hypogonadism (WHO group I anovulation) do not have the threshold levels of endogenous LH required to achieve optimal follicular development and steroidogenesis during therapy with FSH alone. Leptin, a fused protein, or functional fragment thereof alone or in combination with fertility drugs can be used to support ovulation in exchange to LH.

Leptin, a fused protein, or functional fragment thereof can be administrated to patients who did not respond to treatment with fertility drugs such as LH, hCG, clomiphene citrate, GnRH etc in assisted reproductive technologies (ART). Also leptin, a fused protein, or functional fragment thereof can be administered in patients in which the treatment with fertility drugs was inefficient.

Leptin, a fused protein, or functional fragment thereof can be administrated to patients exhibiting hypogonadotropic hypogonadism (HH) who do not have the endogenous threshold of LH required to achieve optimal follicular formation in which hCG administration fail to induce follicle rupture or lutinization.

In addition leptin, a fused protein, or functional fragment thereof can be administered to patients who do not respond adequately to pulsatile GnRH or those patients with pituitary disease.

A method is provided to support ovulation in women which are not responsive to LH and hCG treatment or in which administration of these gonadotropins increase the risk of ovarian hyperstimulation syndrome or polycistic ovary syndrome.

Also provided are pharmaceutical compositions prepared for administration of leptin by mixing leptin, a fused protein, or functional fragment thereof, with physiologically acceptable carriers, and/or stabilizers and/or excipients, and prepared in dosage form, e.g., by lyophilization in dosage vials.

Further provided are pharmaceutical compositions comprising a pharmaceutically acceptable carrier and leptin, a fused protein, and/or functional fragment thereof in the treatment of female infertility.

The pharmaceutical compositions may comprise a pharmaceutically acceptable carrier, leptin, a fusion protein, or functional fragment thereof and optionally further including one or more fertility drugs.

### EXAMPLES

### Example 1

### Leptin induces follicular development in antide-treated ob/ob mice and in prepubertal C57BL/6 mice.

The infertility of female leptin-deficient *ob*/*ob* mice can be completely corrected by long-term leptin treatment, resulting in ovulation and pregnancy and partiturition (Chehab et al. 1996).

Later it was discovered that leptin induces the GnRH release from the Hypothalamus needed for the liberation of FSH and LH from the Pituitary glands that is required for ovulation (Yu et al. 1997).

The effect of leptin in follicular development was studied in *ob*/*ob* mice that were treated with the GnRH antagonist antide (Phillips et al. 1988). It is therefore expected that following administration of leptin to antide treated mice, as opposed to non-treated mice, release of LH or FSH from the pituitary gland will not occur.

8-10 week-old obese C57B1-*ob*/*ob* female mice were all injected (time 0) with antide (1.25 µg/g body weight). Mice were divided into several groups. A control group was injected with saline only. A second group was injected with murine leptin (ip,2x5 µg/g body weight purchased from R&D systems) at times 0 and 9 h. A third group was injected with pregnant mare serum gonadotropins (PMSG, 3 IU/mice, sc). A fourth group received a combination of both PMSG and leptin. Mice were sacrificed at 9, 24, and 48 and 72 h following the first treatment, the ovaries were excised, paraffin-fixed and stained with hematoxylin-eosin to visualize follicular and ovarian growth.

As expected, ovarian sections of control hypogonadal *ob*/*ob* mice that were treated only with antide showed only early stages of follicular growth such as follicles at the primary stage, including small antral follicles (not shown). In contrast, in mice treated with PMSG, which activates the FSH receptors, large antral follicles were formed at 72 h. Surprisingly, treatment of the *ob*/*ob* mice with leptin instead of PMSG also induced rapid follicular growth in the antide-treated *ob*/*ob* mice , manifested by large follicles, which could be observed as early as 9 h after initiation of the leptin tereatment. This result shows that on top of its role as inducer of GnRH, leptin stimulates follicular development by a GnRH-independent pathway. When *ob*/*ob* mice were concomitantly treated with PMSG and leptin, an interstitial cell growth and further ovarian growth were observed after 72 h. More surprisingly, corpora lutea were also seen at 72 h in the ovaries of *ob*/*ob* mice that were treated with PMSG at time 0 and leptin instead of hCG at 48 h (not shown).

Since similar responses were observed in antide-treated prepubertal C57BL/6 mice, it is concluded that leptin may assume the role of hCG in inducing follicular development.
Thus, these results show that the action of leptin directly mediates follicular development.

### Example 2

**Leptin induces ovulation in antide-treated *ob*/*ob and C57Bl* mice and prepubertal C57BL/6 mice.**

The detection of corpora lutea in ovaries of mice treated with PMSG followed by leptin (see previous example) led us to test the possibility that leptin could replace hCG as an inducer of ovulation.

In rodents, ovulation stimulation protocols are based on either a super-ovulatory dose of PMSG (a source of FSH, 40 IU) alone, a non-super-ovulatory dose of PMSG (3-4 IU) in combination with another gonadotropin (3-4 IU LH or hCG) or a continuous sub-cutaneous infusion of purified FSH preparation in combination with luteinizing hormone (LH) or human chronic gonadotropin over a 72-h period (hCG, Leveille et al 1989).

In the present example, ovulation was studied in *ob*/*ob* mice and in prepubertal C57BL/6 mice. All mice were treated with antide to prevent induction of LH by leptin. Ovulation was achieved in control mice by a combination of a non-super-ovulatory dose of PMSG and hCG. In a second group of mice, hCG was replaced by leptin. Following the various treatments, mice were sacrificed and oocytes were collected from the ampoule at 72 hours from the first injection and counted.

Control *ob*/*ob* mice were injected with PMSG (3 IU/animal, purchased from Tiferet Hacarmel pharmacy, Tel Yizhack, Israel) and after 48 h with hCG, the mimicker of LH (human hCG Gibco, USA 5 IU/animal). Similarly a group of *ob*/*ob* mice were injected with PMSG (3 IU/animal) and after 48 h with leptin (2x5 µg/g weight). 24 hours following the hCG or leptin treatment (72 hours from the PMSG treatment) the animals were sacrificed, the ovaries extracted and the formation of corpora lutea, a marker of ovulation, was examined by staining of ovarian sections. Ovulation was monitored by counting oocytes in the oviducts under the microscope. The results summarized in Table 1 show that ovulation was observed only in mice receiving, either hCG or leptin.

Thus, leptin appears to be able to replace hCG as inducer of ovulation in both *ob*/*ob* and in normal premature C57B1 mice.

Therefore, in addition to its capacity as inducer of follicular growth (see Example 1), leptin, like hCG and LH, directly triggers ovulation.

**Table 1**

| Treatment (hours) | Oocyte count in *ob*/*ob* mice | Oocyte count in C57BL/6 mice 5 |
|---|---|---|
| Antide (72) | N.D | 0 (n=5) |
| Antide+PMSG (72) | 0 (n=2) | 0 10 (n=4) |
| Antide+PMSG (48) then Leptin (24) | (range 1-6 oocytes per mouse) 3 out of 5 mice ovulated | (2-7) 7 out of 7 mice ovulated |
| Antide+PMSG (48) then hCG (24) | (1-6) 3 out 3 mice ovulated | (12-28) 15 3 out of 3 mice ovulated |

### Example 3

### LH is not induced by leptin in antide-treated mice.

Leptin induces the GnRH release from the Hypothalamus, which in turn releases FSH and LH from the Pituitary glands (Yu et al. 1997). To test if the follicle development and ovulation induced in the antide-treated C57BL mice and *oblob* mice by leptin is mediated by LH release, serum LH as well as progesterone, which is an LH-induced marker was measured. Measurement of plasma murine LH (mLH) and progesterone were carried out with specific radioimmunoassays. Progesterone was determined in mouse sera by RIA (Kohen 1975). LH was determined in mouse by a specific RIA, obtained trough the National Hormone & Pituitary Program, harbor-UCLA Medical Center (Torrance, California). The levels of mLH were below 0.2 ng/ml in all cases (Table 2). The levels of LH remained low throughout the follicular growth and ovulation in leptin-treated mice. However, it is difficult to detect the LH surge even in positive controls. Therefore, measurement of the LH-induced serum progesterone serve as a more reliable marker of circulating LH. The results of serum progesterone are shown in Figure 1. The results revealed lack of significant progesterone synthesis in leptin-treated mice. In contrast, mice treated with the LH-equivalent hCG had increased serum progesterone.

The difference between hCG and leptin treatment was also noticed in the appearance of the uterus. During the pro-estrous and estrus days of cycle the mouse uterus exhibits maximal distension due to high serum estradiol. This appearance was clearly observed in PMSG-treated mice (not shown). However, the distension and hyperemia subside following the LH surge [Tienhoven,1968], as well as follow administration of hCG, reflecting the decrease in serum estradiol and the concomital increase in serum progesterone. In contrast, the uterus of *ob*/*ob* mice that ovulated upon leptin administration remained hyperemic, exhibiting maximal distension (not shown). In fact, treatment with leptin rendered the uterus hyperemic even in the absence of PMSG. These same uterine features, were also observed in antide-treated C57BL/6 mice. These difference in the uterine physiology and appearance further support the notion that the leptin-induced ovulation is independent of LH activity.

These results demonstrate that leptin induces follicle development and ovulation in antide-treated mice by an LH-independent mechanism

**Table 2**

| **Treatment** | **Serum LH (ng/ml)** |
|---|---|
| Control (no antide) 72 h | 0.04 |
| Antide 72 h | 0.04 ± 0.01 (n=2) |
| Antide+leptin 9 h | 0.05 |
| Antide+leptin 24 h | 0.06 ± 0.01 (n=3) |
| Antide+leptin 48 h | 0.07± 0.04 (n=2) |
| Antide+leptin 72 h | 0.04 ± 0.01 (n=3) |
| Antide+PMSG 60 h | 0.2 |
| Antide+PMSG 48 h then leptin 12 h | 0.17 ± 0 (n=2) |
| Antide+PMSG 72 h | 0.09 ±0.01 (n=2) |
| Antide+PMSG 48 h then leptin 24 h | 0.057 ± 0.01 (n=8) |
| Antide +PMSG 48 h then hCG 24 h | N.D. |

### Example 4

### Leptin induces rupture-associated proteases in pre-ovulatory follicles

The pre-ovulatory surge of LH induces ovulation by several mechanisms, including activation of enzymes that weaken the follicular wall to facilitate the extrusion of follicular contents. These enzymes include a disintegrin and metalloproteinase with thrombospondin-like motif (ADAMTS-1) and cathepsin L (Robker, 2000).

To test whether leptin can induce the expression of ADAMTS-1 and cathepsin L by acting directly on the ovary, in vitro experiments were preformed on pre-ovulatory follicles obtained from 21 days old C57BL/6 mice treated with PMSG (3 IU/animal) for 48h. Pre-ovulatory follicles were removed, cultured and further treated for 17 h in the absence or presence of either leptin (250 ng/ml) or hCG (1 IU). Total RNA was isolated from the follicles and subjected to quantitative RT-PCR using the LightCycler^{™} (Roche diagnostics). The primers used for the detection of murine ADAMTS-1 transcripts were:
Forward primer (SEQID NO:1) : 5'CAGTACCAGACCTTGTGCAGACCTT 3',
Reverse primer (SEQID NO:2): 5' CACACCTCACTGCTTACTGGTTTGA 3'

The primers used for the detection of murine Cathepsin L transcripts were:
Forward primer (SEQID NO:3): 5' TGACACAGGGTTCGTGGATA 3'
Reverse primer (SEQID NO:4): 5' ACCGCTACCCATCAATTCAC 3'

The RNAs levels of ADAMTS-1 and cathepsin L detected in agarose gel electrophoresis of the PCR products were normalized to the RNAs levels of murine ribosomal protein L19 (forward primer (SEQID NO:5): 5' CTGAAGGTCAAAGGGAATGTG 3', reverse primer (SEQID NO:6): 5' GGACAGAGTCTTGATGATCTC 3').

The results shown in Figure 2 demonstrate that a significant induction of ADAMTS-1 was detected at 17 h in leptin (250 ng/ml) or hCG (1IU) -treated follicles. Marginal induction of Cathepsin L was noticed, but it was not statistically significant (not shown).

### REFERENCES

Ahima RS, Prabakaran D, Mantzoros C, Qu D, Lowell B', Maratos-Flier E, Flier JS. "Role of leptin in the neuroendocrine response to fasting." Nature 1996 Jul 18;382(6588):250-2.

Amsterdam, A. and S. Rotmensch, Structure-function relationships during granulosa cell differentiation. Endocr. Rev.,1987. 8: p. 309-337.

Barash, I.A., et al., Leptin is a metabolic signal to the reproductive system. Endocrinology, 1996. 137: p. 3144-3147.

Barkan D, Jia H, Dantes A, Vardimon L, Amsterdam A, Rubinstein M.Leptin modulates the glucocorticoid-induced ovarian steroidogenesis. Endocrinology 1999 Apr;140(4):1731-8.

Brannian JD, Schmidt SM, Kreger DO, Hansen KA. "Baseline non-fasting serum leptin concentration to body mass index ratio is predictive of IVF outcomes." Hum Reprod 2001 Sep;16(9):1819-26.

Chappel SC, Howles C. Reevaluation of the roles of luteinizing hormone and follicle stimulating hormone in the ovulatory process. Hum Reprod 1991 Oct; 6(9):1206-1212.

Chehab, F.F., M.E. Lim, and R. Lu, Correction of the sterility defect in homozygous obese female mice by treatment with the human recombinant leptin. Nat-Genet, 1996. 12(3): p. 318-320.

Dick C Shoot, Herjan JT, Bernadette MJL, Steven WJ, Phillippe Bouchard, Bart Fauser. Human recombinant follicle-stimulating hormone induces growth of preovulatory follicles without concomitant increase in androgen and estrogen biosynthesis in a woman with isolated gonadotrphin deficiency. J Clin Endocrinol Metab 1992; 74(6): 1471-1473.

Erickson JC, Hollopeter G, Palmiter RD. "Attenuation of the obesity syndrome of ob/ob mice by the loss of neuropeptide Y." Science 1996 Dec 6;274(5293):1704-7.

Erickson GF, Wang C. Fsh induction and functional lh receptors in granulosa cells cultured in a chemically defined medium. Nature 1979; 279: 336.

Erickson GF, Magoffin DA. The ovarian androgen producing cells: A review of structure / function relationship. Endocrine Rev 1985; 6: 371-399.

Farooqi, I.S., et al., Effects of recombinant leptin therapy in a child with congenital leptin deficiency. N Engl J Med, 1999. 341(12): p. 879-84.

Greisen S, Ledet T, Moller N, Jorgensen JO, Christiansen JS, Petersen K, Ovesen P. "Effects of leptin on basal and FSH stimulated steroidogenesis in human granulosa luteal cells."Acta Obstet Gynecol Scand 2000 Nov;79(11):931-5.

Hardie, L., et al., Circulating leptin in women: a longitudinal study in the menstrual cycle and during pregnancy. Clin Endocrinol (Oxf), 1997.47(1): p.101-6.

Hiller SG, Smith CD, Whitelaw PF, Miro F, Howles CM. Gonadotrophin control of follicular function. Horm Res 1995; 43: 216-223.

Hillier SG : Regulatory functions for inhibin and activin in human ovaries. J Endocrinol 1991; 131: 171-175.

Hwa, JJ., et al., Leptin increases energy expenditure and selectively promotes fat metabolism in ob/ob mice. Amer J Physiol-Regul Integr C, 1997. 41(4): p. R1204-R1209.

Imani B, Eijkemans MJ, de Jong FH, Payne NN, Bouchard P, Giudice LC, Fauser BC. "Free androgen index and leptin are the most prominent endocrine predictors of ovarian response during clomiphene citrate induction of ovulation in normogonadotropic oligoamenorrheic infertility." J Clin Endocrinol Metab 2000 Feb;85(2):676-82.

Karlsson C, Lindell K, Svensson E, Bergh C, Lind P, Billig H, Carlsson LM, Carlsson B."Expression of functional leptin receptors in the human ovary." J Clin Endocrinol Metab 1997 Dec;82(12):4144-8.

Kessel B, Liu YX, Jia XC, Hsueh AJW. Autocrine role of estrogens in the augmentation of luteinizing hormone receptor formation in cultured rat granulosa cells. Biol Reprod 1985; 32: 1038-50.

Leveille MC, Armstrong DT. "Preimplantation embryo development and serum steroid levels in immature rats induced to ovulate or superovulate with pregnant mares' serum gonadotropin injection or follicle-stimulating hormone infusions." Gamete Res 1989 May;23(1):127-38.

Mannaerts B, Fauser B, Lahlou N, Harlin J, Shoham Z, Bennik HC, Bouchard P. Serum hormone concentrations during treatment with multiple rising doses of recombinant follicle stimulating hormone (Puregon) in men with hypogonadotropic hypogonadism. Fertil Steril 1996 Feb; 65(2): 406-410.

Messinis, I.E., et al., Leptin concentrations in the follicular phase of spontaneous cycles and cycles superovulated with follicle stimulating hormone. Hum Reprod, 1998. 13(5): p. 1152-6.

Mounzih, K., R. Lu, and F.F. Chehab, Leptin treatment rescues the sterility of genetically obese ob/ob males. Endocrinology, 1997.138(3): p. 1190-1193.
Muzzin, P., et al., Correction of obesity and diabetes in genetically obese mice by leptin gene therapy. Proc Natl Acad Sci USA, 1996. 93(25): p. 14804-14808.
Nagatani, S., et al., Evidence for GnRH regulation by leptin: leptin administration prevents reduced pulsatile LH secretion during fasting. Neuroendocrinology, 1998. 67(6): p. 370-6.

Phillips, A., et al., Evaluation of the anaphylactoid activity of a new LHRH antagonist. Life Sci, 1988.43(11): p. 883-8.

Remohi J, Simon C, Pellicer A, Bonilla F. Reproduccion humana. Cap. 1,2,3,4,5,6 ,16,17,24,25,26. McGraw Hill. Interamericana de Espana, S.A. 1996/1997.

Robker RL, Russell DL, Espey LL, Lydon JP, O'Malley BW, Richards JS. "Progesterone-regulated genes in the ovulation process: ADAMTS-1 and cathepsin L proteases." Proc Natl Acad Sci U S A 2000 Apr 25;97(9):4689-94.

Schoot DC, Harlin J, Shaham Z, Mannaerts BM, Lahlou N, Bouchard P, Bennik HJ, Fauser BC. Recombinant human follicle-stimulating hormone and ovarian Response in gonadotrophin-deficient women. Hum Reprod 1994 Jul; 9(7): 1237-1242.

Schwartz, M.W., et al., Specificity of leptin action on elevated blood glucose levels and hypothalamic neuropeptide Y gene expression in ob/ob mice. Diabetes, 1996. 45(4): p.

Shimizu, H., et al., Estrogen increases in vivo leptin production in rats and human subjects. J Endocrinol, 1997. 154(2): p. 285-92.

Simon JA, Danforth DR, Hutchinson JS, Hodgen GD. Characterization of recombinant DNA-derived human luteinizing hormone in vitro and in vivo. Efficacy in ovulation induction and corpus luteum support. Jama 1988; 259: 3290-3295.

Smyth CD, Miro F, Howles CM, Hillier SG. Effect of luteinizing hormone on follicle stimulating hormone-activated paracrine signaling in rat ovary. Hum Reprod 1995, Jan; 10(1): 33-39.

Strobel, A., et al., A leptin missense mutation associated with hypogonadism and morbid obesity. Nat Genet, 1998.18(3): p. 213-5.

The European Recombinant Human Lh Study Group. Recombinant human luteinizing hormone to support recombinant human follicle stimulating hormone-induced follicular development in Lh and Fsh deficient anovulatory women: A dose finding study. J Clin Endocrinol Metab 1998 May; (83)5: 1507-1514.

Tienhoven V (1968) Reproductive physiology of vertebrates (WB. Saunders company).

Unkila-Kallio L, Andersson S, Koistinen HA, Karonen SL, Ylikorkala O, Tiitinen A."Leptin during assisted reproductive cycles: the effect of ovarian stimulation and of very early pregnancy." Hum Reprod 2001 Apr;16(4):657-62.

Weston AM, Zelinski-Wooten MB, Hutchison JS, Stouffer RL, Wolf DP. Deve-lopmental potential of embryos produced by in-vitrofertilization from gonadotro-phin-realising hormone antagonist-treated macaques stimulated with recombinant human follicle stimulating hormone alone or in combination with luteinizing hormone. Hum Reprod 1996 Mar; 11(3): 608-613.

Yu WH, Kimura M, Walczewska A, Karanth S, McCann SM. "Role of leptin in hypothalamic-pituitary function." Proc Natl Acad Sci U S A 1997 Sep 30;94(20):11108.

Zachow RJ, Magoffin DA. "Direct intraovarian effects of leptin: impairment of the synergistic action of insulin-like growth factor-I on follicle-stimulating hormone-dependent estradiol-17 beta production by rat ovarian granulosa cells." Endocrinology 1997 Feb;138(2):847-50.

Zhang, Y., et al., Positional cloning of the mouse obese gene and its human homologue. Nature, 1994. 372: p. 425-432.

Zelinski-Wooten MB, Hutchinson JS, Aladin Chandrasekher Y, Wolf DP, Stoufer RL. Administration of human luteinizing hormone to macaques following follicular development: further titration of lh surge requirements for ovulatory changes in primate follicles. J Clin Endocrinl Metab 1992; 75: 502-507.

## Claims

1. Use of leptin, a fused protein or functional fragment thereof having substantially similar activity to leptin in the manufacture of a medicament for inducing ovulation in the treatment of female infertility.

2. The use according to claim 1, for inducing follicular development.

3. The use according to anyone of claims 1 to 2, for inducing follicular rupture.

4. The use according to anyone of claims 1 to 3, for inducing lutenization.

5. The use according to anyone of claims 1 to 4, wherein the female is not responsive to treatment with fertility drugs.

6. The use according to claim 5, wherein the female is not responsive to hCG treatment.

7. The use according to claim 5, wherein the female is not responsive to LH treatment.

8. The use according to claim 5, wherein the female is not responsive to GnRH treatment.

9. The use according to claim 5, wherein the female is not responsive to clomiphene citrate treatment.

10. The use according to claim 5, wherein the female is not responsive to HMG treatment.

11. The use according to claim 5, wherein the female is not responsive to progesterone treatment.

12. The use according to anyone of the preceding claims, wherein the medicament further comprises a fertility drug selected from hCG, LH, GnRH, clomiphene citrate, HMG and progesterone.

13. The use of leptin according to anyone of claims 1 to 4, in a female in which the administration of LH or hCG increases the risk of acquiring ovarian hyperstimulation syndrome or polycystic ovary syndrome.

14. The use of leptin according to anyone of claims 1 to 4, for treatment of a female with pituitary disease.

15. The use of leptin according to anyone of claims 1 to 14, for treatment in assisted reproductive technologies.

16. The use of leptin according to claim 15 for *in vitro* fertilization treatment.

## Patentansprüche

1. Verwendung von Leptin, einem Fusionsprotein oder funktionellen Fragment davon, das eine im Wesentlichen ähnliche Aktivität wie Leptin besitzt, zur Herstellung eines Medikaments zur Ovulationsinduzierung bei der Behandlung weiblicher Unfruchtbarkeit.

2. Verwendung nach Anspruch 1, um die Follikelentwicklung zu induzieren.

3. Verwendung nach einem der Ansprüche 1 oder 2, um das Reißen des Follikels zu induzieren.

4. Verwendung nach einem der Ansprüche 1 bis 3 um die Luteinisierung zu induzieren.

5. Verwendung nach einem der Ansprüche 1 bis 4, wobei die Frau gegenüber einer Behandlung mit Fertilitätsarzneimitteln nicht reagiert.

6. Verwendung nach Anspruch 5, wobei die Frau gegenüber einer Behandlung mit hCG nicht reagiert.

7. Verwendung nach Anspruch 5, wobei die Frau gegenüber einer Behandlung mit LH nicht reagiert.

8. Verwendung nach Anspruch 5, wobei die Frau gegenüber einer Behandlung mit GnRH nicht reagiert.

9. Verwendung nach Anspruch 5, wobei die Frau gegenüber einer Behandlung mit Clomiphencitrat nicht reagiert.

10. Verwendung nach Anspruch 5, wobei die Frau gegenüber einer Behandlung mit HMG nicht reagiert.

11. Verwendung nach Anspruch 5, wobei die Frau gegenüber einer Behandlung mit Progesteron nicht reagiert.

12. Verwendung nach einem der vorhergehenden Ansprüche, wobei das Medikament ferner ein Fertilitätsarzneimittel ausgewählt aus hCG, LH, GnRH, Clomiphencitrat, HMG und Progesteron enthält.

13. Verwendung von Leptin nach einem der Ansprüche 1 bis 4 an einer Frau, bei der die Verabreichung von LH oder hCG das Risiko erhöht, das ovarielle Hyperstimulationssyndrom oder das polycystische Ovarialsyndrom zu erwerben.

14. Verwendung von Leptin nach einem der Ansprüche 1 bis 4 zur Behandlung einer Frau mit einer Hypophysenkrankheit.

15. Verwendung von Leptin nach einem der Ansprüche 1 bis 14 zur Behandlung in unterstützten Reproduktionstechniken.

16. Verwendung von Leptin nach Anspruch 15 zur *In vitro*-Fertilisations-Behandlung.

## Revendications

1. Emploi de la leptine, ou d'une protéine de fusion de leptine ou d'un fragment fonctionnel de leptine, doté d'une activité sensiblement similaire à celle d'une leptine, dans la fabrication d'un médicament conçu pour induire l'ovulation dans le traitement de la stérilité féminine.

2. Emploi conforme à la revendication 1, en vue d'induire le développement folliculaire.

3. Emploi conforme à la revendication 1 ou 2, en vue d'induire la rupture folliculaire.

4. Emploi conforme à l'une des revendications 1 à 3, en vue d'induire la lutéinisation.

5. Emploi conforme à l'une des revendications 1 à 4, dans le cas d'une femme qui ne répond pas à un traitement par des médicaments combattant la stérilité.

6. Emploi conforme à la revendication 5, dans le cas d'une femme qui ne répond pas à un traitement par de la gonadotrophine chorionique humaine (hCG).

7. Emploi conforme à la revendication 5, dans le cas d'une femme qui ne répond pas à un traitement par de l'hormone lutéinisante (LH).

8. Emploi conforme à la revendication 5, dans le cas d'une femme qui ne répond pas à un traitement par de la gonadolibérine (GnRH).

9. Emploi conforme à la revendication 5, dans le cas d'une femme qui ne répond pas à un traitement par du citrate de clomiphène.

10. Emploi conforme à la revendication 5, dans le cas d'une femme qui ne répond pas à un traitement par de la gonadotrophine ménopausique humaine (HMG).

11. Emploi conforme à la revendication 5, dans le cas d'une femme qui ne répond pas à un traitement par de la progestérone.

12. Emploi conforme à l'une des revendications précédentes, ledit médicament comprenant en outre un médicament combattant la stérilité, choisi parmi les hormones hCG, LH, GnRH et HMG, le citrate de clomiphène et la progestérone.

13. Emploi de la leptine, conforme à l'une des revendications 1 à 4, dans le cas d'une femme chez laquelle l'administration d'hormone LH ou hCG augmenterait le risque de développement d'un syndrome d'hyperstimulation des ovaires ou d'une polykystose ovarienne.

14. Emploi de la leptine, conforme à l'une des revendications 1 à 4, en vue de traiter une femme atteinte d'une maladie hypophysaire.

15. Emploi de la leptine, conforme à l'une des revendications 1 à 14, en vue d'un traitement par une technique de reproduction assistée.

16. Emploi de la leptine, conforme à la revendication 15, en vue d'un traitement de fertilisation *in vitro.*


### SEQUENCE LISTING

<110> Yeda Researcha and Development Co. Ltd.
   BARKAN, Dalit
   DEKEL, Nava
   RUBINSTEIN, Menachem
   HURGIN, Vladimir
<120> The use of Leptin in fertility
<130> 522
<150> 146655
<151> 2001-11-21
<160> 6
<170> PatentIn version 3.1
<210> 1
<211> 25
<212> DNA
<213> Murinae gen. sp.
<400> 1
   cagtaccaga ccttgtgcag acctt 25
<210> 2
<211> 25
<212> DNA
<213> Murinae gen. sp.
<400> 2
   cacacctcac tgcttactgg tttga 25
<210> 3
<211> 20
<212> DNA
<213> Murinae gen. sp.
<400> 3
   tgacacaggg ttcgtggata 20
<210> 4
<211> 20
<212> DNA
<213> Murinae gen. sp.
<400> 4
   accgctaccc atcaattcac 20
<210> 5
<211> 21
<212> DNA
<213> Murinae gen. sp.
<400> 5
   ctgaaggtca aagggaatgt g 21
<210> 6
<211> 21
<212> DNA
<213> Murinae gen. sp.
<400> 6
   ggacagagtc ttgatgatct c 21